(19) **Europäisches Patentamt**

**European Patent Office**

**Office européen des brevets**

(11) **EP 1 229 938 B1**

(12) **EUROPEAN PATENT SPECIFICATION**

(45) Date of publication and mention
of the grant of the patent:
**13.09.2006 Bulletin 2006/37**

(51) Int Cl.:
*A61K 31/7088* (2006.01)　　*A61K 31/4745* (2006.01)
*A61P 35/00* (2006.01)

(21) Application number: **00977058.7**

(22) Date of filing: **08.11.2000**

(86) International application number:
**PCT/US2000/030687**

(87) International publication number:
**WO 2001/034093 (17.05.2001 Gazette 2001/20)**

(54) **POTENTIATION OF THE ACTIVITY OF SN-38 PRODRUGS**

POTENZIERUNG DER WIRKSAMKEIT VON SN-38-PRODROGEN

POTENTIALISATION DE L'ACTIVITE DE PROMEDICAMENTS DE SN-38

(84) Designated Contracting States:
**AT BE CH CY DE DK ES FI FR GB GR IE IT LI LU MC NL PT SE TR**

(30) Priority: **09.11.1999 US 164182 P**

(43) Date of publication of application:
**14.08.2002 Bulletin 2002/33**

(73) Proprietor: **HYBRIDON, INC.
Cambridge,
Massachusetts 02139 (US)**

(72) Inventor: **AGRAWAL, Sudhir
Shrewsbury, MA 01545 (US)**

(74) Representative: **Lee, Nicholas John et al
Kilburn & Strode,
20 Red Lion Street
London WC1R 4PJ (GB)**

(56) References cited:
　**WO-A-96/17626**　　　　**WO-A-99/38964**

• **P.M.POTTER E.A.: "Isolation and partial characterization of a cDNA encoding a rabbit liver carboxylesterase that activates the prodrug irinotecan (CPT-11)" CANCER RESEARCH, vol. 58, no. 12, 1998, pages 2646-2651, XP002919026**

**Description**

The invention relates to the therapeutic use of prodrugs. More particularly, the invention relates to methods for potentiating the efficacy of prodrugs.

Many potential pharmaceutical agents fail to be used therapeutically due to excessive toxicity or limited bioavailability. In some instances, these limiting factors can be ameliorated by modifying the pharmaceutical agent to create a prodrug. The prodrug is then converted by the body into the pharmaceutically active substance.

International application number WO98/07734 discloses the manufacture of oligonucleotide prodrugs having ester or amide modifications that cover a non-bridging oxygen of the phosphodiester linkage. Kuhn, Oncology, Supplement No. 6, 39-42 (1998) discloses that CPT-11 (Camptosar) is an antineoplastic prodrug that is converted by carboxylesterase activity in the liver and other tissues to the active agent SN-38. Cerosimo, The Annals of Pharmacotherapy 32:1324-1333 (1998) teaches that the parent compound of CPT-11, camptothecin, was unable to be developed as a pharmaceutical due to severe toxicity.

Due to the presence of carboxylesterases and amidases in the liver and other tissues, the ability to make prodrugs which have added ester or amide groups is a generalizable phenomenon. However, these compounds generally retain at least some of the toxicity of the parent compound, due to rapid hydrolysis of the prodrug. Kuhn, *supra,* discloses that SN-38, the active metabolite of CPT-11 still causes diarrhea, which is the limiting toxicity of the parent compound, camptothecin.

Thus, there is a need for methods to administer prodrugs in a manner that maximizes their efficacy while avoiding significant toxicity. Ideally, such methods should affect the manner in which the body processes prodrugs, and thus would be applicable to a broad range of prodrugs.

In a first aspect, the invention provides one or more products containing an oligonucleotide phosphorothioate or phosphorodithioate and an SN-38 prodrug as a combined preparation for simultaneous, separate or sequential use for statistically significantly potentiating the activity of the SN-38 prodrug without producing significant side effects, wherein the oligonucleotide phosphorothioate or phosphorodithioate is not an oligonucleotide having two 5'and four 3'2'-0-methylribonucleosides and having the sequence 5'-UGACACCTGTTCTCACUCAC-3'.

In a second aspect, the invention provides one ore more products containing an oligonucleotide phosphorothioate or phosphorodithioate and an SN-38 prodrug as a combined preparation for simultaneous, separate or sequential use for statistically significantly potentiating the activity of the SN-38 prodrug without producing significant side effects, wherein the oligonucleotide phosphorothioate or phosphorodithioate is for administration before the prodrug.

In a third aspect, the invention provides one or more products containing an oligonucleotide phosphorothioate or phosphorodithioate and an SN-38 prodrug as a combined preparation for simultaneous, separate or sequential use for statistically significantly potentiating the activity of the SN-38 prodrug without producing significant side effects, wherein the SN-38 prodrug is present in an amount that would not be therapeutically effective in the absence of the one or more products.

In a fourth aspect, present the invention provides the use of an oligonucleotide phosphorothioate or phosphorodithioate in the manufacture of a medicament for statistically significantly potentiating the activity of an SN-38 prodrug without producing significant side effects, wherein the oligonucleotide phosphorothioate or phosphorodithioate is not an oligonucleotide having two 5'and four 3'2'-0-methylribonucleosides and having the sequence 5'-UGACACCTGTTCTCACUCAC-3'.

In a fifth aspect, the present invention provides the use of an oligonucleotide phosphorothioate or phosphorodithioate in the manufacture of a medicament for statistically significantly potentiating the activity of an SN-38 prodrug without producing significant side effects, wherein the oligonucleotide phosphorothioate or phosphorodithioate is for administration before the prodrug.

In a sixth aspect, the present invention provides the use of an oligonucleotide phosphorothioate or phosphorodithioate in the manufacture of a medicament for statistically significantly potentiating the activity of an SN-38 prodrug without producing significant side effects, wherein the SN-38 prodrug is present in an amount that would not be therapeutically effective in the absence of the oligonucleotide phosphorothioate or phosphorodithioate.

In the present invention, the prodrug may be an ester or an amide of an active compound. The active compound may be an anticancer drug, which may be Camptosar. The oligonucleotide may comprise a 2'-O-substituted ribonucleoside. The 2'-O-substituted ribonucleoside may be selected from 2'-O-methyl ribonucleosides and 2'-O-methoxyethoxy ribonucleosides.

The third and sixth aspects of the invention are particularly useful where the toxicity of the prodrug or active compound is dose-limiting. Thus, the third and sixth aspects of the invention can increase the therapeutic index for the prodrug.

The invention provides for the administration of SN-38 prodrugs in a manner that maximizes their efficacy, and thus allows lower, less toxic dosages to be used. The invention acts through a variety of mechanisms that modulate the ability of the body to process the prodrug to the active compound and its ability to clear either the prodrug or the active compound, and are thus applicable to a broad range of SN-38 prodrugs.

## BRIEF DESCRIPTION OF THE DRAWINGS

Figure 1 shows survival times for mice in the HCT116 study using Oligo 1.

Figure 2 shows survival times for mice in the HCT116 study using Oligo 2.

Figure 3 shows a Kaplan-Meier survival plot for mice in the HCT116 study using Oligo 1.

Figur 4 shows a Kaplan-Meier survival plot for mice in the HCT116 study using Oligo 2.

Figure 5 shows the time of administration of oligonucleotide effect on survival times for mice.

Figure 6 shows the efficacy of oral administration of oligonucleotides reflected in survival times for mice.

The invention relates to the therapeutic use of SN-38 prodrugs. More particularly, the invention relates to potentiating the efficacy of SN-38 prodrugs.

As used herein, a "prodrug" is a compound comprising an active compound covalently linked to another moiety by a cleavable linkage, wherein the pharmacological activity of the active compound is greater than the pharmacological activity of the prodrug, and wherein the active compound is produced in the body by cleavage of the cleavable linkage. An "active compound" is a molecule having a pharmacological activity. A "pharmacological activity" is an activity that is useful in the treatment of one or more disease or disease symptom. A "moiety" is a chemical group or structure. A "cleavable linkage" is a covalent bond that can be cleaved by an enzymatic activity in the body. The term "without producing significant side effects" means that any signs or symptoms of toxicity that are observed in the presence of the oligonucleotide phosphorothioate or phosphorodithioate are not greater than those observed in the absence of the oligonucleotide phosphorothioate or phosphorodithioate to an extent that would preclude the combination of the prodrug and the oligonucleotide phosphorothioate or phosphorodithioate from obtaining regulatory approval. The term "co-administration" is intended to include treatment regimens in which either the prodrug or the oligonucleotide phosphorothioate or phosphorodithioate is continued after the cessation of the other agent.

Preferred SN-38 prodrugs include amides and esters of active compounds. Such active compounds include, without limitation, anticancer chemotherapeutics, anti-inflammatory agents, antiinfective agents, antiviral agents and cardiovascular drugs. Numerous prodrugs are well known in the art (see, e.g., *Singh et al., J. Sci. Ind. Res.* 55: 497-510 (1996)). Specific nonlimiting examples of preferred prodrugs include Camptosar ((7-ethyl-10-(-4-piperidinol)-1-piperidnocarbonyloxy-camptothecin; CPT-11) and Camptosar analogs and foscarnate. The moiety that is cleaved from the prodrug may preferably be selected from esters and alpha-acyloxyalkyl esters (for carboxy functionalities); amides, esters, carbonate esters, phosphate esters, ethers and alpha-acyloxyalkyl ethers (for hydroxyl functionalities); thioesters, alpha-acyloxyalkyl thioesters and disulfides (for sulfhydryl functionalities); ketals, imines, enol esters, oxazoladines, and thiazolidines (for carbonyl functionalities); amides, carbamates, imines enamines N-Mannich bases, and N-acyloxyalkoxycarbonyl derivatives (for amino functionalities); N-acyloxyalkyl derivatives (for quarternary amino functionalities); N-sulphonyl imidates (for ester or sulfonamido functionalities); N-Mannich bases (for NH-acidic functionalities); and N-acyloxyalkyl derivatives (for heterocyclic amino functionalities).

For the purposes of the invention, the term "oligonucleotide" includes polymers of two or more deoxyribonucleotide, or any modified nucleoside, including 2'-halo-nucleosides, 2'-O-substituted ribonucleosides, 3'-O-substituted nucleosides, deazanucleosides or any combination thereof. Such monomers may be coupled to each other by any of the numerous known internucleoside linkages. In certain preferred embodiments, these internucleoside linkages may be phosphodiester, phosphotriester, phosphorothioate, or phosphoramidate linkages, or combinations thereof. The linkages may be in any configuration, including without limitation 5'-3', 5'-2', 5'-5'; 3'-3'; 3'-5', 2'-5' or any combination thereof. The term "oligonucleotide" also encompasses such polymers having chemically modified bases or sugars and/or having additional substituents, including without limitation lipophilic groups, cholesterol, folic acid, intercalating agents, diamines and adamantane. Oligonucleotides may also be formulated, *e.g.;* in cyclodextrins and/or liposomes. For purposes of the invention the term "2'-O-substituted" and "3'-O-substituted" mean, respectively, substitution of the 2' or 3' position of the pentose moiety with a halogen (preferably Cl, Br, or F), or an -O-lower alkyl group containing 1-6 saturated or unsaturated carbon atoms, or with an -O-aryl or allyl group having 2-6 carbon atoms, wherein such alkyl, aryl or allyl group may be unsubstituted or maybe substituted, e.g., with halo, hydroxy, trifluoromethyl, cyano, nitro, acyl, acyloxy, alkoxy, carboxyl, carbalkoxyl, or amino groups; or such 2' substitution may be with a hydroxy group (to produce a ribonucleoside), an amino or a halo group, but not with a 2'-H group. In certain preferred embodiments, the 2'-O-substituted ribonucleoside is selected from 2'-O-methyl ribonucleosides and 2'-O-methoxyethoxy ribonucleosides. In certain preferred embodiments, the 3'-O-substituted ribonucleoside is selected from 3'-O-methyl ribonucleosides and 3'-O-methoxyethoxy ribonucleosides. In certain embodiments all nucleosides may be 2'-O-substituted, preferably 2'-O-alkyl. Oligonucleotides used in the invention also include double stranded oligonucleotides, including hairpin oligonucleotides, as well as cyclic oligonucleotides.

In certain preferred embodiments, an oligonucleotide for use in the invention may be complementary to an endogenous or exogenous nucleic acid sequence, preferably a nucleic acid that is involved in a disease. The term "complementary" means having the ability to hybridize to a genomic region, a gene, or an RNA transcript thereof under physiological conditions. Such hybridization is ordinarily the result of base-specific hydrogen bonding between complementary strands, preferably to form Watson-Crick or Hoogsteen base pairs, although other modes of hydrogen bonding, as well as base stacking can also lead to hybridization. As a practical matter, such hybridization can be inferred from the observation of specific gene expression inhibition. The nucleic acid sequence to which the modified oligonucleotide sequence is complementary will depend upon the biological effect that is sought to be modified. In certain particularly preferred embodiments the oligonucleotide is complementary to a gene selected from mdm-2, PKA, PKC, raf-kinase, bcl-2, H-ras, c-myc, DNA methyltransferase, histone deacetylase and VEGF. In certain preferred embodiments Of the second, third, fifth and sixth aspects of the invention, such an oligonucleotide has the sequence 5'-UGACACCTGTTCTCACUCAC-3'. However, in the first and fourth aspects, oligonucleotides having this sequence are specifically excluded, and in some preferred embodiments oligonucleotides that are complementary to the mdm-2 gene are specifically excluded. In certain embodiments, antisense oligonucleotides are specifically excluded, as the oligonucleotides used in the invention are capable of potentiating the activity of prodrugs in a sequence independent manner.

Oligonucleotides in antisense embodiments are preferably from about 13 to about 100 nucleotides in length, more preferably from about 15 to about 50, and most preferably from about 15 to about 35. Oligonucleotides in non-antisense embodiments can be within these ranges, but can also preferably be from about 5 to about 15 nucleotides in length. Preferably, oligonucleotides used in the invention contain one or more modified internucleoside linkage and may optionally contain either deoxyribonucleosides, ribonucleosides or 2'-O-substituted ribonucleosides, or any combination thereof. Particularly preferred antisense oligonucleotides according to this aspect of the invention include mixed backbone oligonucleotides, including chimeric oligonucleotides and hybrid oligonucleotides.

For purposes of the invention, a "mixed backbone oligonucleotide" is an oligonucleotide having more than one type of backbone substituent, *e.g.*, differences in the sugar and/or internucleoside linkages among the various nucleosides comprising the oligonucleotide.

For purposes of the invention, a "chimeric oligonucleotide" refers to an oligonucleotide having more than one type of internucleoside linkage: One preferred embodiment of such a chimeric oligonucleotide is a chimeric oligonucleotide comprising a phosphorothioate, phosphodiester or phosphorodithioate region, preferably comprising from about 2 to about 12 nucleotides, and a nonionic region, preferably an alkylphosphonate or alkylphosphonothioate region. Preferably, such chimeric oligonucleotides contain at least three consecutive internucleoside linkages selected from phosphodiester and phosphorothioate linkages, or combinations thereof.

For purposes of the invention, a "hybrid oligonucleotide" refers to an oligonucleotide having more than one type of nucleoside. One preferred embodiment of such a hybrid oligonucleotide comprises a ribonucleotide or 2'-O-substituted ribonucleotide region, preferably comprising from about 2 to about 12 2'-O-substituted nucleotides, and a deoxyribonucleotide region. Preferably, such a hybrid oligonucleotide will contain at least three consecutive deoxyribonucleosides and will also contain ribonucleosides, 2'-O-substituted ribonucleosides, or combinations thereof. In a preferred embodiment, the deoxynucleotide region is flanked on either side by a 2'-O-substituted region. In one particularly preferred embodiment, the 2'-O-substituted regions are 2'-O-methyl regions, most preferably having four 2'-O-methyl nucleosides. In certain preferred embodiments the entire backbone of the oligonucleotide is a phosphorothioate backbone. Particularly preferred hybrid oligonucleotides comprise one or more 2'-O-methyl ribonucleoside or 2'-O-methoxyethoxy ribonucleoside.

The synthesis of oligonucleotides can now be routinely accomplished. See e.g., *Methods in Molecular Biology, Vol 20: Protocols for Oligonucleotides and Analogs* pp. 165-189 (S. Agrawal, Ed., Humana Press, 1993); *Oligonucleotides and Analogues: A Practical Approach,* pp. 87-108 (F. Eckstein, Ed., 1991); and Uhlmann and Peyman, *supra.* Agrawal and Iyer, *Curr. Op. in Biotech.* 6: 12 (1995); and *Antisense Research and Applications* (Crooke and Lebleu, Eds., CRC Press, Boca Raton, 1993).

Without wishing to be bound by theory, the potentiation of the prodrug is believed to involve one or more of the following mechanisms:

- modulation of the retention time of the prodrug in the liver and other tissues, including tumor tissue
- competition with cleavage enzymes or other hepatic enzymes, e.g., carboxylesterases, amidases, or other esterases
- competition with transport factors from the liver, e.g., cMOAT for CPT-11

- competition for binding of serum proteins
- competition with binding of endothelial cell walls
- competition for covalent modification, e.g., glucouronidation
- slowing hydrolysis of the prodrug so that active metabolite is continuously released into the blood circulation
- stabilization of the active form of the drug, e.g., lactone formation for CPT-11.

Any of these mechanisms may benefit by saturation of the system with the oligonucleotide phosphorothioate or phosphorodithioate prior to administration of the prodrug.

The following examples are intended to further illustrate certain particularly preferred embodiments of the invention and are not intended to limit the scope of the invention. Oligo 1 does not fall within the scope of the first and fourth aspects of the invention.

Example 1

<u>Treatment of colon cancer tumor-bearing mice</u>

Female NCr-nude mice, 6-8 weeks of age, were fed *ad libitum* water (reverse osmosis, 0.17% Cl) and an autoclaved standard rodent diet (NIH31) of 18% protein; 5% fat, 5% fiber, 8% ash and 3% minerals. Mice were housed in microisolators on a 12 hour light cycle at 22°C in 40-60% humidity. Mice were implanted subcutaneously in the flank with 1 $mm^3$ HCT-116 human colon carcinoma fragments in the flank. Tumors were monitored twice weekly initially, then daily as the tumors reached approximately 100 mg in weight. When the tumors reached a weight between 40-221 mg (calculated weight), the animals were pair-matched into the various treatment groups. Estimated tumor weight was determined according to the equation: tumor weight =

$$\frac{w^2 \times l}{2}$$

where w = width and l = length in mm of a HCT-116 tumor. Phosphorothioate oligonucleotides having 2'-O-methylribonucleosides at the 2 terminal 5' positions and 4 terminal 3' positions (Oligo 1), or the 4 terminal 5' and 3' positions (Oligo 2) were prepared according to standard procedures and dissolved in neutral buffered saline. Oligo 1 had the sequence 5'-UGACACCTGTTCTCACUCAC-3' (complementary to mdm-2), and the aequence of Oligo 2 was 5'-UCGCACCCATCTCTCTCCUUC-3' (complementary to the HIV-1 gag gene). Camptosar was purchased from Pharmacia & Upjohn.

Animals were pair-matched on Day 1 into 12 groups with 9 mice per group. Oligo or Oligo 2 was administered i.p. at 10mg/kg doses on a 5/2/5/2/5/2/5 schedule (i.e., five days dosing, two days rest, repeat). Camptosar was administered i.v. at doses of 25 or 50 mg/kg once a week for 3 weeks. For combined treatments, 5 or 10 mg/kg of Oligo 1 was administered i.p. with Camptosar at 25 mg/kg, or 10 mg/kg Oligo 1 was administered was administered i.p. with 50 mg/kg Camptosar. Oligo 2 was administered at a dose of 10 mg/kg i.p. with 25 or 50 mg/kg Camptosar. Control animals were treated with vehicle i.p. on a 5/2/5/2/5/2/5 schedule. The study was terminated on day 56.

Results were determined using the tumor growth delay (TGD) endpoint method. Each mouse was euthanized when its HCT-116 tumor reached a weight of 1.5 g; this was taken as a cancer death. Mean Day of Survival (MDS) was calculated for each group based upon the calculated day of death according to: Time to end point (calculated) = Time to exceed endpoint (observed) minus

$$\frac{\frac{Wt_2 - \text{endpoint weight}}{Wt_2 - Wt_1}}{D_2 - D_1}$$

where Time to exceed endpoint (observed) is the number of days it takes for each tumor to grow past the endpoint (cut-off) weight (mouse is euthanized), $D_2$ is the day that the mouse is euthanized, $D_1$ is the last day of caliper measurement before the tumor reaches endpoint, $Wt_2$ is tumor weight (mg) on $D_2$, $Wt_1$ is tumor weight (mg) on $D_1$, and Endpoint weight is the predetermined "cut-off" tumor weight for the model being used. For statistical analysis, the unpaired t-test and Mann-Whitney U test (analyzing means and medians respectively) were used to determine the statistical significance of differences in survival times between groups. These analyses were conducted at a p level of 0.05 (two-tailed) using Prism (GraphPad) version 3.0.

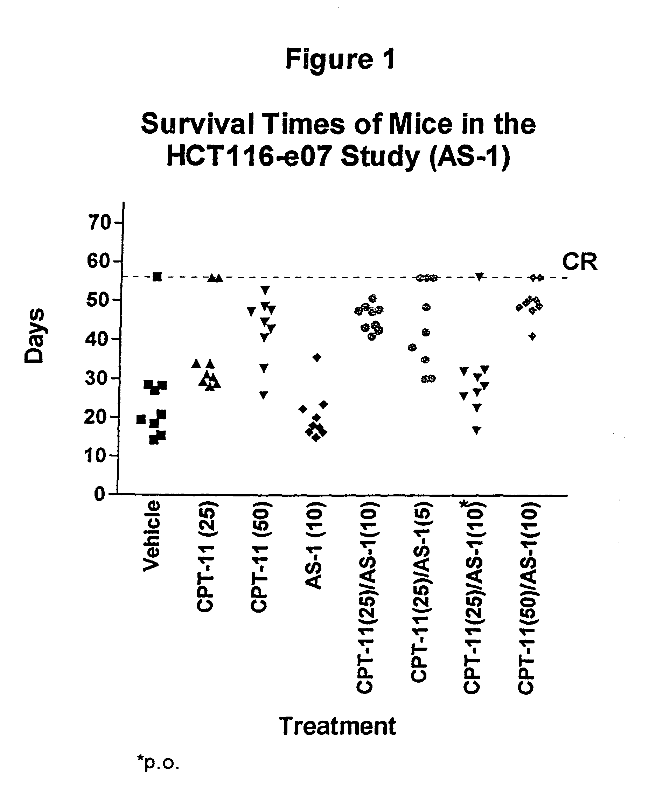

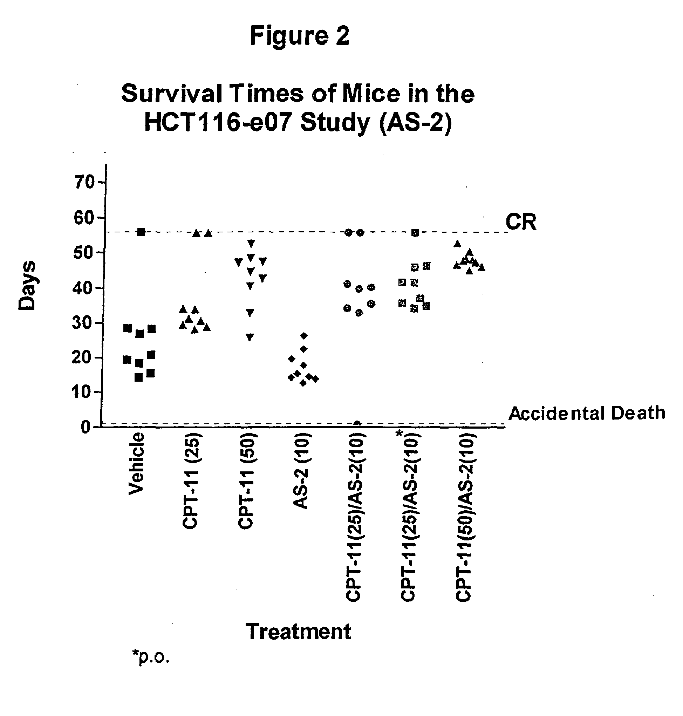

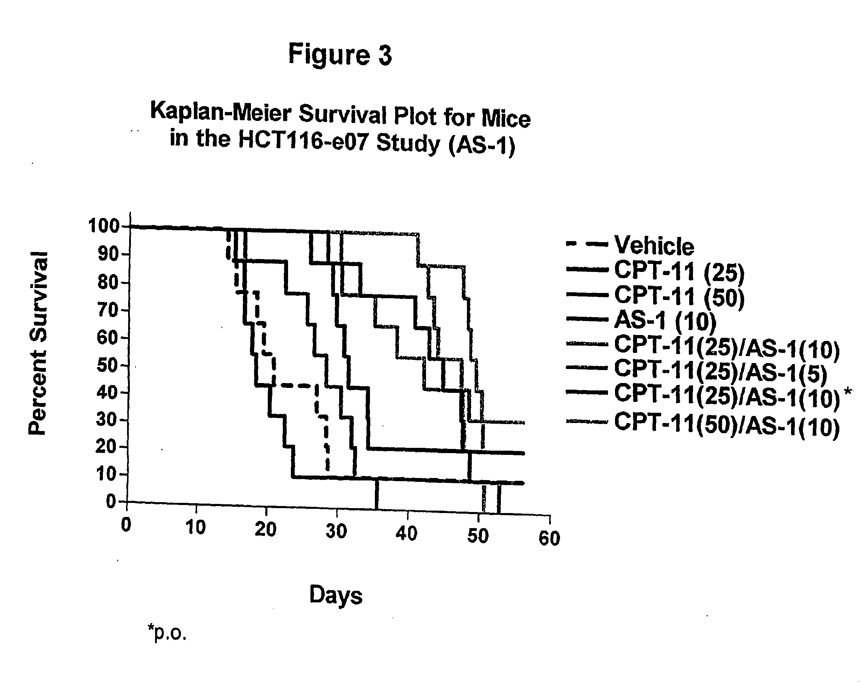

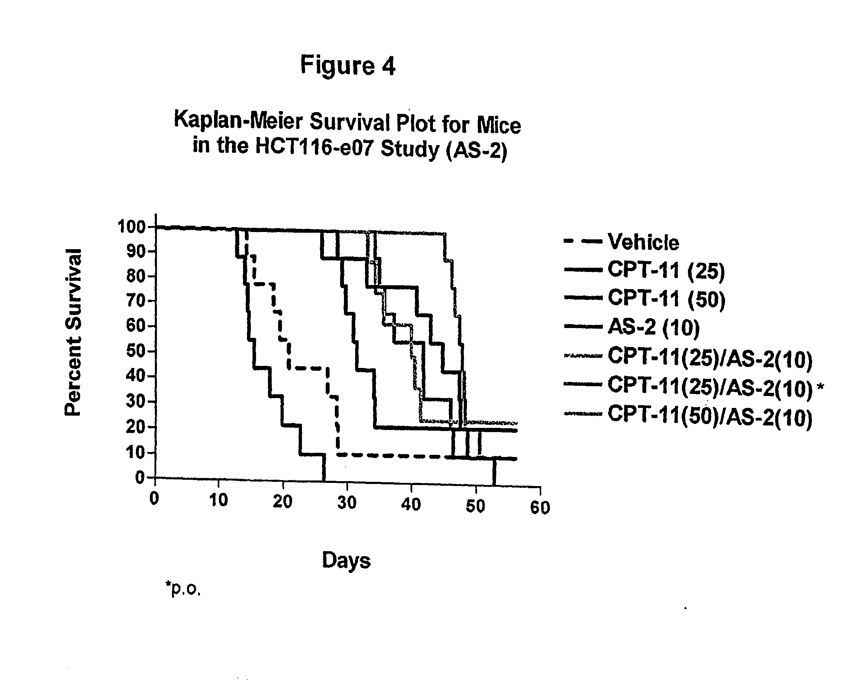

Of the 9 vehicle control mice, 8 had tumors reaching the 1.5 g endpoint with an MDS value of 21.5 days. One tumor regressed completely, presumably due to poor tumor take. Camptosar at 25 mg/kg produced an MDS value of 31.1 days, and at 50 mg/kg, 42.6 days. Neither Oligo 1 nor Oligo 2 alone produced any prolongation of MDS. However, administration of 10 mg/kg Oligo 1 with 25 mg/kg Camptosar extended MDS over vehicle controls by 24.4 days, and over mice treated with 25 mg/kg Camptosar alone by 14.8 days. Each of these extensions is statistically significant ($p < 0.0001$; unpaired t-test). Mice treated with 5 mg/kg Oligo 1 and 25 mg/kg achieved an MDS value of 37.4 days, which was statistically significant over vehicle controls ($p < 0.0005$; unpaired t-test) and over mice treated with 25 mg/kg Camptosar alone ($p < 0.046$; unpaired t-test). Administration of 10 mg/kg Oligo 2 i.p. with 25 mg/kg Camptosar produced an MDS value of 39.7 days, which is statistically significant over vehicle controls ($p < 0.0001$; unpaired t-test) and over mice treated with 25 mg/kg Camptosar alone ($p < 0.0009$; unpaired t-test). Administration of 10 mg/kg Oligo 2 i.p. with 50 mg/kg Camptosar produced an MDS value of 42.6 days, which trends toward statistical significance over mice treated with 50 mg/kg Camptosar alone ($p < 0.08$; unpaired t-test). These results demonstrate that both Oligo 1 and Oligo 2 can potentiate the activity of Camptosar efficacy in a statistically significant and dose-dependent manner, and that at least part of this effect is independent of oligonucleotide sequence. The results of these studies are summarized in Figures 1-4.

Comparison of the potentiation of Camptosar efficacy by Oligo 1 against potentiation of Camptosar efficacy by Oligo 2 shows that there is a statistically significant difference in favor of Oligo 1 ($p < 0.0074$; unpaired t-test). It is believed that this difference may arise from an antisense effect of Oligo 1 on expression of the mdm-1 oncogene to which it is complementary.

Example 2

Treatment of pancreatic cancer tumor-bearing mice

To test whether the differences between Oligo 1 and Oligo 2 resulted from an antisense effect by Oligo 1, similar studies were conducted in a mouse model for pancreatic cancer (Panc 1 tumor). The Panc 1 tumor has a mutant (nonfunctional) p53 gene. Since antisense effects against mdm-1 are believed to work primarily by upregulating p53 expression, Oligo 1 should not produce an antisense specific effect in this model. However, it is possible that mdm-2 targeted oligonucleotides in p53 mutant cell lines may work by a mechanism independent of p53.

The study was carried out as described in Example 1, except that Panc-1 tumor was used, 4 groups of 10 mice each were used, Oligo 1 and Oligo 2 (in this case, 5'-UCCCACCTATTCTTACUCCC-3', with two 5'-terminal 2'-O-methylribonucleosides and four 3'-terminal 2'-O-methylribonucleosides) were given at doses of 20 mg/kg, Camptosar was given at 100 mg/kg, tumor "cut-off" was 1.2 g, and the study was terminated on Day 67.

Both Oligo 1 and Oligo 2 showed statistically significant potentiation of Camptosar efficacy ($p < 0.05$; unpaired t-test). The potentiating effects of Oligo 1 and Oligo 2, compared with each other, were statistically indistinguishable. These results demonstrate that oligonucleotides produce a statistically significant potentiating effect on Camptosar that is independent of the sequence of the oligonucleotide. Moreover, in these studies treatment with Camptosar alone was not statistically significantly better than treatment with vehicle. Thus, these results demonstrate that oligonucleotides can potentiate the effectiveness of Camptosar such that an otherwise sub-therapeutic dosage of Camptosar becomes therapeutically effective.

Example 3

Effect of timing and route of oligonucleotide administration

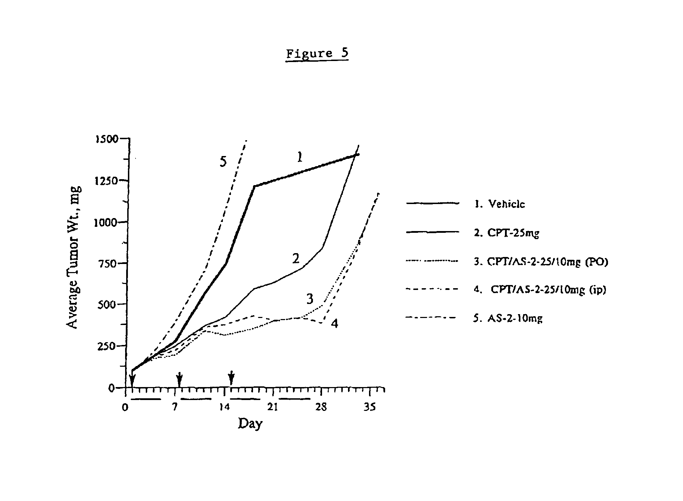

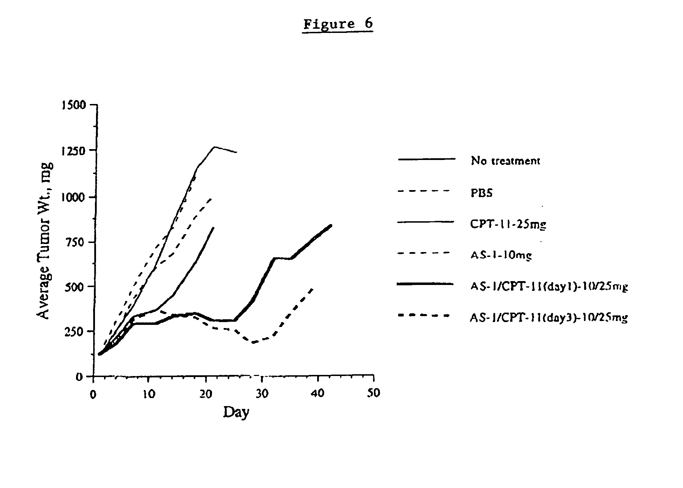

The study of Example 1 was repeated, but the oligonucleotide was administered initially on day 1 and Captosar was not administered initially until day 3. Surprisingly, this schedule of administration was even more effective (see Figure 5). Also, the study of Example 1 was repeated, but the oligonucleotide was administered orally. This route of administration was equally effective (see Figure 6).

**Claims**

1. One or more products containing an oligonucleotide phosphorothioate or phosphorodithioate and an SN-38 prodrug as a combined preparation for simultaneous, separate or sequential use for statistically significantly potentiating the activity of the SN-38 prodrug without producing significant side effects, wherein the oligonucleotide phosphorothioate or phosphorodithioate is not an oligonucleotide having two 5'and four 3'2'-0-methylribonucleosides and having the sequence 5'-UGACACCTGTTCTCACUCAC-3'.

2. One ore more products containing an oligonucleotide phosphorothioate or phosphorodithioate and an SN-38 prodrug as a combined preparation for simultaneous, separate or sequential use for statistically significantly potentiating the activity of the SN-38 prodrug without producing significant side effects, wherein the oligonucleotide phosphorothioate or phosphorodithioate is for administration before the prodrug.

3. One or more products containing an oligonucleotide phosphorothioate or phosphorodithioate and an SN-38 prodrug as a combined preparation for simultaneous, separate or sequential use for statistically significantly potentiating the activity of the SN-38 prodrug without producing significant side effects, wherein the SN-38prodrug is present in an amount that would not be therapeutically effective in the absence of the one or more products.

4. Products according to claim 1, 2 or 3, wherein the SN-38 prodrug is an ester or an amide of an active compound.

5. Products according to claim 4, wherein the active compound is an anticancer drug.

6. Products according to claim 5, wherein the SN-38 prodrug is Camptosar.

7. Products according to any preceding claim, wherein the oligonucleotide comprises a 2'-O-substituted ribonucleoside.

8. Products according to claim 7, wherein the 2'-O-substituted ribonucleoside is selected from 2'-O-methyl ribonucleosides and 2'-O-methoxyethoxy ribonucleosides.

9. The use of an oligonucleotide phosphorothioate or phosphorodithioate in the manufacture of a medicament for statistically significantly potentiating the activity of an SN-38 prodrug without producing significant side effects, wherein the oligonucleotide phosphorothioate or phosphorodithioate is not an oligonucleotide having two 5'and four 3'2'-0-methylribonucleosides and having the sequence 5'-UGACACCTGTTCTCACUCAC-3'.

10. The use of an oligonucleotide phosphorothioate or phosphorodithioate in the manufacture of a medicament for statistically significantly potentiating the activity of an SN-38 prodrug without producing significant side effects, wherein the oligonucleotide phosphorothioate or phosphorodithioate is for administration before the prodrug.

11. The use of an oligonucleotide phosphorothioate or phosphorodithioate in the manufacture of a medicament for statistically significantly potentiating the activity of an SN-38 prodrug without producing significant side effects, wherein the SN-38 prodrug is present in an amount that would not be therapeutically effective in the absence of the oligonucleotide phosphorothioate or phosphorodithioate.

12. The use according to claim 9, 10 or 11, wherein the prodrug is an ester or an amide of an active compound.

13. The use according to claim 12, wherein the active compound is an anticancer drug.

14. The use according to claim 13, wherein the SN-38 prodrug is Camptosar.

15. The use according to any one of claims 9 to 14, wherein the oligonucleotide comprises a 2'-O-substituted ribonucleoside.

16. The use according to claim 15, wherein the 2'-O-substituted ribonucleoside is selected from 2'-O-methyl ribonucleosides and 2'-O-methoxyethoxy ribonucleosides.

**Patentansprüche**

1. Ein oder mehrere Produkt(e), die ein Oligonukleotid-Phosphorothioat oder Phosphorodithioat und ein SN-38-Pro-Pharmakon als eine kombinierte Zubereitung enhalten, für die simultane, getrennte oder sequentielle Verwendung für die statistisch signifikante Wirksammachung der Aktivität des SN-38-Pro-Pharmakon ohne signifikante Nebenwirkung zu erzeugen, wobei das Oligonukleotid-Phosphorothioat oder Phosphorodithioat kein Oligonukleotid ist, das zwei 5' und vier 3'2'-0-Methylribonukleoside und die Sequenz 5'-UGACACCTGTTCTCACUCAC-3'.

**2.** Ein oder mehrere Produkt(e), die ein Oligonukleotid-Phosphorothioat oder Phosphorodithioat und ein SN-38-Pro-Pharmakon als eine kombinierte Zubereitung enhalten, für die simultane, getrennte oder sequentielle Verwendung für die statistisch signifikante Wirksammachung der Aktivität des SN-38-Pro-Pharmakon ohne signifikante Nebenwirkung zu erzeugen, wobei das Oligonukleotid-Phosphorothioat oder Phosphorodithioat für die Verabreichung vor dem Pro-Pharmakon ist.

**3.** Ein oder mehrere Produkt(e), die ein Oligonukleotid-Phosphorothioat oder Phosphorodithioat und ein SN-38-Pro-Pharmakon als eine kombinierte Zubereitung enhalten, für die simultane, getrennte oder sequentielle Verwendung für die statistisch signifikante Wirksammachung der Aktivität des SN-38-Pro-Pharmakon ohne signifikante Nebenwirkung zu erzeugen, wobei das SN-38-Pro-Pharmakon in einer Menge vorhanden ist, die in der Abwesenheit von dem einen oder mehreren Produkten therapeutisch nicht wirksam sein würde.

**4.** Produkte nach Anspruch 1, 2 oder 3, wobei das SN-38-Pro-Pharmakon ein Ester oder ein Amid des Wirkstoffes ist.

**5.** Produkte nach Anspruch 4, wobei der Wirkstoff ein Antikrebs-Arzneimittel ist.

**6.** Produkte nach Anspruch 5, wobei das SN-38-Pro-Pharmakon Camptosar ist.

**7.** Produkte nach einem der vorangehenden Ansprüche, wobei das Oligonukleotid ein 2'-O-substitutiertes Ribonukleosid aufweist.

**8.** Produkte nach Anspruch 7, wobei das 2'-O-substitutierte Ribonukleosid aus 2'O-Methylribonukleosiden und 2'-O-Methoxyethoxyriobnukleosiden ausgewählt ist.

**9.** Verwendung eines Oligonukleotid-Phosphorothioats oder Phosphorodithioats zum Herstellen eines Medikamentes für die statistisch signifikante Wirksammachung der Aktivität des SN-38-Pro-Pharmakon ohne signifikante Nebenwirkung zu erzeugen, wobei das Oligonukleotid-Phosphorothioat oder Phosphorodithioat kein Oligonukleotid ist, das zwei 5' und vier 3'2'-0-Methylribonukleoside und die Sequenz 5'-UGACACCTGTTCTCACUCAC-3'.

**10.** Verwendung eines Oligonukleotid-Phosphorothioats oder Phosphorodithioats zum Herstellen eines Medikamentes für die statistisch signifikante Wirksammachung der Aktivität des SN-38-Pro-Pharmakon ohne signifikante Nebenwirkung zu erzeugen, wobei das Oligonukleotid-Phosphorothioat oder Phosphorodithioat für die Verabreichung vor dem Pro-Pharmakon ist.

**11.** Verwendung eines Oligonukleotid-Phosphorothioats oder Phosphorodithioats zum Herstellen eines Medikamentes für die statistisch signifikante Wirksammachung der Aktivität des SN-38-Pro-Pharmakon ohne signifikante Nebenwirkung zu erzeugen, wobei das SN-38-Pro-Pharmakon in einer Menge vorhanden ist, die in der Abwesenheit von dem einen oder mehreren Produkten therapeutisch nicht wirksam sein würde.

**12.** Verwendung nach Anspruch 9, 10, oder 11, wobei das SN-38-Pro-Pharmakon ein Ester oder ein Amid des Wirkstoffes ist.

**13.** Produkte nach Anspruch 12, wobei der Wirkstoff ein Antikrebs-Arzneimittel ist.

**14.** Produkte nach Anspruch 13, wobei das SN-38-Pro-Pharmakon Camptosar ist.

**15.** Verwendung nach einem der Ansprüche 9 bis 14, wobei das Oligonukleotid ein 2'-O-substitutiertes Ribonukleosid aufweist.

**16.** Verwendung nach Anspruch 15, wobei das 2'-O-substitutierte Ribonukleosid aus 2'O-Methylribonukleosiden und 2'-O-Methoxyethoxyriobnukleosiden ausgewählt ist.

**Revendications**

**1.** Un ou plusieurs produits contenant un oligonucléotide à phosphorothioate ou phosphorodithioate et un promédicament SN-38 sous forme d'une préparation combinée pour une utilisation simultanée, distincte ou séquentielle pour potentialiser de manière statistiquement significative l'activité du promédicament SN-38 sans produire d'effets secondaires significatifs, où l'oligonucléotide à phosphorothioate ou phosphorodithioate n'est pas un oligonucléotide présentant deux 5' et quatre 3',2'-O-méthylribonucléosides et présentant la séquence 5'-UGACACCTGTTCTCACUCAC-3'.

2. Un ou plusieurs produits contenant un oligonucléotide à phosphorothioate ou phosphorodithioate et un promédicament SN-38 sous forme d'une préparation combinée pour une utilisation simultanée, distincte ou séquentielle pour potentialiser de manière statistiquement significative l'activité du promédicament SN-38 sans produire d'effets secondaires significatifs, où l'oligonucléotide à phosphorothioate ou phosphorodithioate est destiné à une administration avant le promédicament.

3. Un ou plusieurs produits contenant un oligonucléotide à phosphorothioate ou phosphorodithioate et un promédicament SN-38 sous forme d'une préparation combinée pour une utilisation simultanée, distincte ou séquentielle pour potentialiser de manière statistiquement significative l'activité du promédicament SN-38 sans produire d'effets secondaires significatifs, où le promédicament SN-38 est présent en une quantité qui ne serait pas efficace sur le plan thérapeutique en l'absence du produit ou des plusieurs produits.

4. Produits suivant la revendication 1, 2 ou 3, dans lesquels le promédicament SN-38 est un ester ou un amide d'un composé actif.

5. Produits suivant la revendication 4, dans lesquels le composé actif est un médicament anti-cancer.

6. Produits suivant la revendication 5, dans lesquels le promédicament SN-38 est le Camptosar.

7. Produits suivant l'une quelconque des revendications précédentes, dans lesquels l'oligonucléotide comprend un ribonucléoside à substitution 2'-O.

8. Produits suivant la revendication 7, dans lesquels le ribonucléoside à substitution 2'-O est sélectionné parmi les 2'-O-méthylribonucléosides et les 2'-O-méthoxyéthoxyribonucléosides.

9. Utilisation d'un oligonucléotide à phosphorothioate ou phosphorodithioate dans la fabrication d'un médicament pour potentialiser de manière statistiquement significative l'activité du promédicament SN-38 sans produire d'effets secondaires significatifs, où l'oligonucléotide à phosphorothioate ou phosphorodithioate n'est pas un oligonucléotide présentant deux 5' et quatre 3',2'-O-méthylribonucléosides et présentant la séquence 5'-UGACACCTGTTCTCACUCAC-3'.

10. Utilisation d'un oligonucléotide à phosphorothioate ou phosphorodithioate dans la fabrication d'un médicament pour potentialiser de manière statistiquement significative l'activité du promédicament SN-38 sans produire d'effets secondaires significatifs, où l'oligonucléotide à phosphorothioate ou phosphorodithioate est destiné à une administration avant le promédicament.

11. Utilisation d'un oligonucléotide à phosphorothioate ou phosphorodithioate dans la fabrication d'un médicament pour potentialiser de manière statistiquement significative l'activité du promédicament SN-38 sans produire d'effets secondaires significatifs, où le promédicament SN-38 est présent en une quantité qui ne serait pas efficace sur le plan thérapeutique en l'absence de l'oligonucléotide à phosphorothioate ou phosphorodithioate.

12. Utilisation suivant la revendication 9, 10 ou 11, dans laquelle le promédicament est un ester ou un amide d'un composé actif.

13. Utilisation suivant la revendication 12, dans laquelle le composé actif est un médicament anti-cancer.

14. Utilisation suivant la revendication 13, dans laquelle le promédicament SN-38 est le Camptosar.

15. Utilisation suivant l'une quelconque des revendications 9 à 14, dans laquelle l'oligonucléotide comprend un ribonucléoside à substitution 2'-O.

16. Utilisation suivant la revendication 15, dans laquelle le ribonucléoside à substitution 2'-O est sélectionné parmi les 2'-O-méthylribonucléosides et les 2'-O-méthoxyéthoxyribonucléosides.

# Figure 1

## Survival Times of Mice in the HCT116-e07 Study (AS-1)

# Figure 2

**Survival Times of Mice in the HCT116-e07 Study (AS-2)**

# Figure 3

## Kaplan-Meier Survival Plot for Mice in the HCT116-e07 Study (AS-1)

*p.o.

EP 1 229 938 B1

# Figure 4

## Kaplan-Meier Survival Plot for Mice
## in the HCT116-e07 Study (AS-2)

**Percent Survival** (y-axis: 0, 10, 20, 30, 40, 50, 60, 70, 80, 90, 100)

**Days** (x-axis: 0, 10, 20, 30, 40, 50, 60)

Legend:
- − − Vehicle
- —— CPT-11 (25)
- —— CPT-11 (50)
- —— AS-2 (10)
- ······ CPT-11(25)/AS-2(10)
- —— CPT-11(25)/AS-2(10) *
- ····· CPT-11(50)/AS-2(10)

*p.o.

Figure 5

Figure 6